# EUROPEAN PATENT APPLICATION

(11) **EP 1 884 246 A1**
(43) Date of publication of application: **06.02.2008**
(21) Application number: 06016356.5
(22) Date of filing: 04.08.2006
(51) Int. Cl.: A61K 38/57, A61P 37/00

(54) **Cystatin from nematodes in the treatment of autoimmune or allergic diseases**

(71) Applicant: Humboldt-Universität zu Berlin, D-10099 Berlin (DE); Charité-Universitätsmedizin Berlin, 10117 Berlin (DE)
(72) Inventor: Hartmann, Susanne, 12307 Berlin (DE); Lucius, Richard, 10717 Berlin (DE); Schnöller, Corinna, 10407 Berlin (DE); Hamelmann, Eckard, 12203 Berlin (DE)
(74) Representative: Engelhard, Markus

(57) **Abstract**

The present invention relates to uses of cystatins derived from nematodes and to a method of screening using such cystatin.

SEQUENCE LISTING

<110> Charite Universitätsmedizin Berlin

<120> Uses of cystatin

<130> FB17401

<160> 3

<170> PatentIn version 3.3

<210> 1
<211> 157
<212> PRT
<213> Acanthocheilonema viteae

<400> 1

Met Met Leu Ser Ile Lys Glu Asp Gly Leu Leu Val Val Leu Leu Leu
1               5                   10                  15

Ser Phe Gly Val Thr Thr Val Leu Val Arg Cys Glu Glu Pro Ala Asn
            20                  25                  30

Met Glu Ser Glu Val Gln Ala Pro Asn Leu Leu Gly Gly Trp Gln Glu
        35                  40                  45

Arg Asn Pro Glu Glu Lys Glu Ile Gln Asp Leu Leu Pro Lys Val Leu
    50                  55                  60

Ile Lys Leu Asn Gln Leu Ser Asn Val Glu Tyr His Leu Met Pro Ile
65                  70                  75                  80

Lys Leu Leu Lys Val Ser Ser Gln Val Val Ala Gly Leu Arg Tyr Lys
                85                  90                  95

Met Glu Ile Gln Val Ala Gln Ser Glu Cys Lys Lys Ser Ser Gly Glu
            100                 105                 110

Glu Val Asn Leu Lys Thr Cys Lys Arg Leu Glu Gly His Pro Asp Gln
        115                 120                 125

Ile Ile Thr Leu Glu Ala Trp Glu Lys Ser Trp Glu Asn Phe Leu Gln
    130                 135                 140

Val Lys Ile Leu Glu Lys Lys Glu Val Leu Ser Ser Val
145                 150                 155

<210> 2
<211> 162
<212> PRT
<213> Onchocerca volvulus

<400> 2

Met Leu Thr Ile Lys Asp Gly Thr Leu Leu Ile His Leu Leu Leu Phe
1               5                   10                  15

Ser Val Val Ala Leu Val Gln Leu Gln Gly Ala Lys Ser Ala Arg Ala
            20                  25                  30

Lys Asn Pro Ser Lys Met Glu Ser Lys Thr Gly Glu Asn Gln Asp Arg
        35                  40                  45

Pro Val Leu Leu Gly Gly Trp Glu Asp Arg Asp Pro Lys Asp Glu Glu
    50                  55                  60

Ile Leu Glu Leu Leu Pro Ser Ile Leu Met Lys Val Asn Glu Gln Ser
65                  70                  75                  80

Asn Asp Glu Tyr His Leu Met Pro Ile Lys Leu Leu Lys Val Ser Ser
                85                  90                  95

Gln Val Val Ala Gly Val Lys Tyr Lys Met Asp Val Gln Val Ala Arg
            100                 105                 110

Ser Gln Cys Lys Lys Ser Ser Asn Glu Lys Val Asp Leu Thr Lys Cys
        115                 120                 125

Lys Lys Leu Glu Gly His Pro Glu Lys Val Met Thr Leu Glu Val Trp
    130                 135                 140

Glu Lys Pro Trp Glu Asn Phe Met Arg Val Glu Ile Leu Gly Thr Lys
145                 150                 155                 160

Glu Val

<210> 3
<211> 161
<212> PRT
<213> Brugia malayi

<400> 3

Met Met Ser Thr Met Ser Ile Lys Glu Gly Leu Leu Val Ile Leu Leu
1               5                   10                  15

Ser Leu Phe Leu Phe Asp Thr Thr Ala Leu Ile His Arg Arg Glu Ile
            20                  25                  30

Pro His Met Glu Ser Lys Gly Gln Met Gln Arg Gly Gln Val Leu Leu

        35                  40                  45

Gly Gly Trp Gln Glu Arg Ser Pro Glu Asp Asn Glu Ile Leu Glu Leu
    50                  55                  60

Leu Pro Ser Val Leu Thr Lys Val Asn Gln Gln Ser Asn Asp Glu Tyr
65                  70                  75                  80

His Leu Met Pro Ile Lys Leu Leu Lys Val Ser Ser Gln Val Val Ala
                85                  90                  95

Gly Val Lys Tyr Lys Met Glu Val Gln Val Ala Arg Ser Glu Cys Lys
            100                 105                 110

Lys Ser Ala Ser Glu Gln Val Asn Leu Lys Thr Cys Lys Lys Leu Glu
        115                 120                 125

Gly His Pro Asp Gln Val Met Thr Leu Glu Val Trp Glu Lys Pro Trp
    130                 135                 140

Glu Asp Phe Leu Gln Val Asn Ile Leu Glu Thr Lys Val Leu Ser Ser
145                 150                 155                 160

Val

## Description

The present invention relates to uses of cystatins derived from nematodes and to a method of screening using such cystatin.

Diseases which are characterized by the presence of undesirable inflammation and inflammatory symptoms contribute significant losses of life expectancy and well-being to a large number of humans. Moreover, they represent a significant factor in damage to national economies in that virtually all human beings at some stage in their life are subject to undesirable inflammations. Treatment of inflammatory diseases in the past has been more or less non-specific, based on broad-spectrum immuno-suppressant drugs, such as corticosteroids. Although beneficial, these drugs have significant side effects which limit their use.

More recently, research has identified a number of ways to specifically block known targets in inflammation. The most successful of these approaches so far has been to block TNF alpha, a hormone-like molecule which signals information from one cell to other cells in the body. Such molecules are collectively known as cytokines. Cytokines like TNF alpha play an essential role in normal immunity, but in disease situations their levels are elevated or reduced and they exert deleterious effects on cell function and hence the well-being of the individual. In certain disease situations, such as colitis and rheumatoid arthritis, blockage of TNF alpha has been associated with some benefit in patients. Despite these successes, currently available TNF alpha treatments are ineffective in approximately half of the patients and in most cases are administered with other drugs. They must be given by injection and are often associated with adverse injection side reactions as well as inconvenience, and can be very costly, potentially limiting their availability to many patients.

One subset of inflammatory diseases are allergic diseases. Allergic diseases are characterized by adverse reactions to normally harmless substances, such as dust, pollen, food or mould. The immune systems of people with allergic diseases overreact to these substances. People who are sensitive to such triggering substances have a high amount of IgE in their blood. If small amounts of the triggering substances, such as pollen granules, meet with IgE in the body of a patient, the body overreacts. The immune system tries to fight the substance that is thus recognized as non-self. This results in allergic symptoms, such as swelling, tearing, congestion, sneezing and other symptoms. Examples of allergic conditions are allergic rhinitis, also referred to as "hay fever", asthma, atopic dermatitis, allergic sinusitis, food allergies. Treatment of allergies has focussed on environmental control, pharmacological therapy and allergen immunotherapy. Environmental control involves avoiding exposure to the triggering substances, however, can only be successful to a certain extent. Pharmacological therapy is mostly a symptomatic treatment. Allergen immunotherapy is a way of desensitizing a patient's immune system by improving the way the immune system responds to an allergic trigger. None of these approaches have been overtly successful. Accordingly, there exists a need in the art to improve current therapies for allergic diseases.

Autoimmune diseases are another subgroup of inflammatory diseases and are accompanied by inflammatory symptoms. Autoimmune diseases are characterized by a misguided immune system, wherein the patient's body or parts thereof is attacked by the immune system and thereby damaged. Autoimmune diseases are characterized by the body's immune response being directed against its own tissues causing prolonged inflammation and subsequent tissue destruction. Autoimmune diseases can cause immune-response of cells to attack the linings of joints or specific types of cells within specific tissues, thereby leading to diseases such as rheumatoid arthritis or insulin-dependent diabetes. Autoimmune diseases include, but are not limited to celiac disease, Crohn's disease, pancreatitis, lupus erhythematosus, psoriasis, multiple sclerosis, inflammatory bowel diseases, Sjogren's syndrome, Hashimoto's thyroiditis etc.

Allergic diseases and autoimmune diseases are characterized by a common etiology, in that in both cases, the immune system is misguided and aims at foreign agents as allegedly harmful agents (which they are not) or aims at the organism's own tissues as allegedly foreign agents (which they are not). In both instances, the effect of such misguidance is a long-term damage to the organism's body by inflammatory cells like neutrophils, eosinophils or macrophages that are attracted to the site of injury and activated, which leads to production of toxic molecules and the damage and destruction of the body's own cells. Consequently, therapeutical approaches to both groups of diseases have common elements, and may target at the differentiation, attraction and activation of inflammatory cells.
None of the current therapies for allergic diseases and/or autoimmune diseases are particularly successful. Accordingly, there exists a need in the art to provide for new ways of therapy for allergic and autoimmune diseases. In particular one object of the present invention was to provide for new ways of therapy of allergic diseases of the respiratory organs, such as asthma, hay fever, allergic sinusitis, allergic rhinitis. Furthermore, it was an object of the present invention to provide for ways of therapy for allergic diseases of the gastrointestinal system, such as food allergies, such as celiac disease, lactose intolerance. Moreover, it was an object of the present invention to provide for ways of treating allergic disease of the skin, such as atopic dermatitis, and/or systemic allergic diseases, such as anaphylactic reactions. Moreover, it was an object of the present invention to provide for new ways of treating autoimmune disease of the joints and/or skin and/or internal organs, such as rheumatoid arthritis, psoriasis, lupus erythematosus, multiple sclerosis and inflammatory bowel disease, such as colitis ulcerosa and Crohn's disease.

All these objects are solved by the use of a cystatin derived from a nematode for the manufacture of a medicament for the prevention and/or treatment of an allergic and/ or autoimmune disease in a patient.

In one embodiment said cystatin is derived from a parasitic nematode.

Preferably, said parasitic nematodes is parasitic to humans.

Preferably, said nematode is selected from the group comprising Onchocerca volvulus, Brugia malayi, Wuchereria bancrofti, Loa loa and Acanthocheilonema viteae.

In one embodiment said allergic and/ or autoimmune disease is selected from the group comprising allergic diseases of the respiratory organs, such as asthma, hay fever, allergic sinusitis, allergic rhinitis, of the gastrointestinal system, such as food allergies, of the skin, such as atopic dermatitis, systemic allergic diseases, such as anaphylactic reactions, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

Prefebly, said allergic disease is asthma or hay fever.

In one embodiment said cystatin has a sequence selected from the group comprising SEQ ID NO: SEQ ID NO:1 (Acanthocheilonema viteae cystatin L43053), SEQ ID NO: 2 (Onchocerca volvulus cystatin M37105), SEQ ID NO:3 (Brugia malayi cystatin AF 177193_1 and sequences that are 70% identical, preferably 80% identical, more preferably 90% identical and, most preferably, 99% identical to any of the foregoing.

In one embodiment said cystatin is recombinant cystatin and has been produced by a procaryotic or eucaryotic expression system.

In one embodiment said disease is associated with an increased count of eosinophil blood cells and/or with an increased level of IgE, when compared with a patient not having said disease, and said medicament, upon its administration to said patient, leads to a reduction of said increased count of eosinophil blood cells and/or to a reduction of said increased level of IgE, preferably to a count of eosinophil blood cells and/or to a level of IgE of a healthy individual.

Preferably, said increased count of eosinophil blood cells is > 4% of all white blood cells of a patient or > 360/µl total number of eosinophil blood cells in peripheral blood of a patient, and said increased level of IgE is > 100 kU/l serum level in an adult patient.

In one embodiment said cystatin is administered to said patient as a protein.

In another embodiment said cystatin is administered to said patient as a nucleic acid encoding said cystatin.

In one embodiment said cystatin is administered systemically to said patient, preferably by injection, inhalation and or other incorporation such as ingestion.

In one embodiment said cystatin is administered to said patient intranasally, intrapulmonarily, intraperitoneally, intrathecally, intralesionally, subcutaneously and/or intramuscularly.

In a preferred embodiment said cystatin is administered in combination with another drug selected from the group of anti-inflammatory drugs, such as corticosteroids, non-steroidal anti-inflammatory drugs and/or anti-histamines.

In one embodiment said patient is a mammal, preferably a human being.

In one embodiment said cystatin is used to bind to CD36 receptor.

The objects of the present invention are also solved by a method of screening for a candidate drug useful for the prevention and/or treatment of an allergic and/or autoimmune disease comprising the following steps:
- providing a first group of cells of a type expressing CD36-receptor,
- exposing said cells to a cystatin derived from a nematode, said cystatin and said nematode being defined as in any of claims 1-16,
- detecting and quantitating, as a first signal, the extent of binding between said cystatin and said CD36 receptor,
- providing a second group of cells of the same type as the first group of cells,
- exposing said second group of cells to a candidate compound,
- detecting and quantitating, as a second signal, the extent of binding between said candidate compound and said CD36 receptor,
- comparing said first signal with said second signal, and
- identifying said candidate compound as a candidate drug for the prevention and/or treatment of an allergic and/or autoimmune disease, if the extent of binding quantitated by said second signal is equal to or greater than the extent of binding quantitated by said first signal.

The objects of the present invention are also solved by the use of CD36-receptor in a method of screening for candidate compounds for the prevention and/or treatment of an allergic and/or autoimmune disease is a patient.

As used herein, the term "cystatin" refers to members of a super family of inhibitors of cysteine proteases

A "parasitic nematode" is a nematode that exploits a host organism for meeting its own requirements. This may involve a life of the nematode within the tissue of a host for parts or the entire life-span of the nematode. In preferred embodiments, the host of such parasitic nematode is human.

A sequence that has x% identity to another sequence is a sequence in which x% residues on their respective positions are identical when optimally aligned.

The term "a cystatin derived from a nematode" as used herein, is meant to refer to any cystatin that has the sequence of a cystatin occurring in a nematode. The term is not limited to a specific production method and may include isolation of the cystatin from the nematode or production of the cystatin by other methods, such as recombinant techniques or chemical synthesis. The term "a cystatin derived from a nematode" is also meant to include proteins that have retained a cystatin function despite their amino acid sequence having been mutated in one or several positions by substitutions, insertions or deletions. Techniques for producing such mutated cystatins which nevertheless can be considered as being "derived from a nematode" have been described in, for example, "Proteins, Structures and Molecular Properties" by Thomas E. Creighton, second edition, W. H. Freeman and Co., New York, and are known to someone skilled in the art.

The term "said cystatin is administered in combination with another drug" is meant to include any situation wherein said cystatin is administered concomitantly with, before or after administration of such another drug. The other drug and cystatin may be in the same dosage unit, or they may be administered in separate dosage units. They may be administered by the same route or different routes.

The present inventors have surprisingly found that cystatins from parasitic nematodes are capable of suppressing inflammatory immune reactions in their host. In a number of experiments, the present inventors have been able to demonstrate that cystatins of nematodes can suppress immune reactions and are therefore capable of for example inhibiting the induction of allergic airway hyperreactivity, or the induction of allergic gastrointestinal hyper reactivity. Because of the common etiology between allergic diseases and autoimmune diseases, it can also be reasonably assumed that the cystatins according to the present invention are also useful in the treatment and/or prevention of autoimmune diseases. The inventors could furthermore show that the cystatins according to the present invention have an influence on regulatory T-cells, in that they upregulate and induce regulatory T-cells. Moreover, administration of cystatins according to the present invention leads to a substantial reduction in the count of eosinophil blood cells and a reduction of the levels of allergen-specific immune globulin E (IgE). The administration of cystatins according to the present invention leads to a reduction of these two variables back to approximately "normal" levels, i.e. levels of a healthy individual.

Moreover, the present inventors show that on a molecular basis, the cystatins according to the present invention interact with the CD36-receptor which makes the CD36-receptor a prime target for future drug studies, more specifically in research aimed at finding new therapies for the treatment and/or prevention of allergic diseases and/or autoimmune diseases.

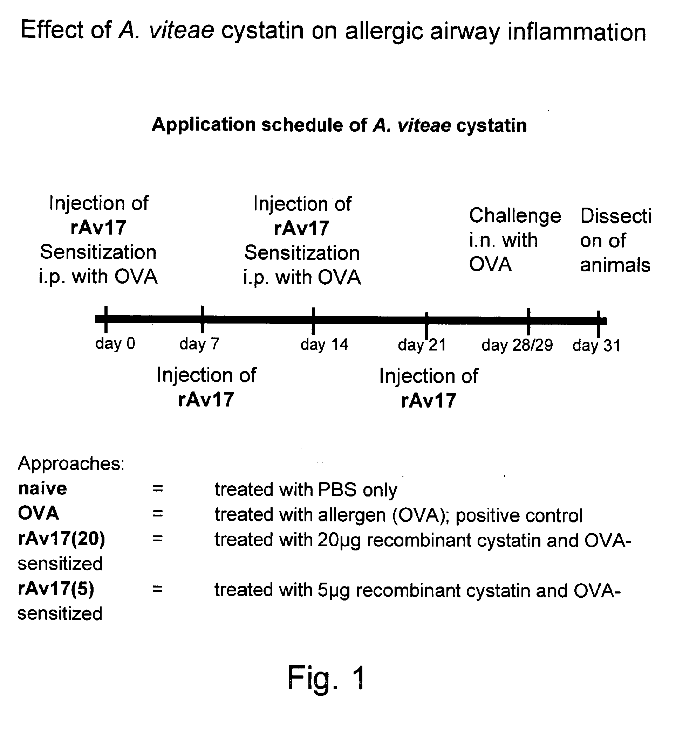

In the following, reference is made to the figures, wherein
figure 1 shows an application schedule of A. viteae cystatin (recombinant Acanthocheilonema viteae cystatin (rAv 17) at two different concentrations (20 µg and 5 µg)),

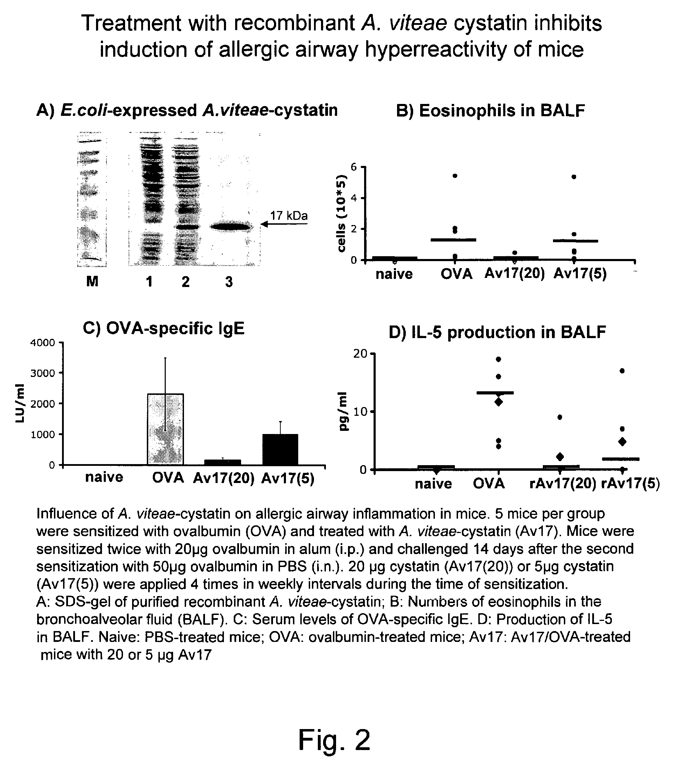

figure 2 shows the influence of A. viteae-cystatin on an animal model for allergic airway inflammation in mice, wherein figure 2a shows an SDS-gel of purified recombinant A. viteae-cystatin, figure 2b shows the numbers of eosinophils in the bronchoalveolar fluid (BALF), figure 2c shows the serum levels of ovalbumine-specific IgE, and figure 2d shows the production of IL-5 in bronchoalveolar fluid, wherein naive means mice treated with PBS (phosphate buffered saline only, OVA means mice treated with ovalbumine, rAv17(20) or (5) means rAv17/OVA treated mice with 20 or 5 µg of rAv17, respectively,
figure 3 shows the influence of cystatins according to the present invention on regulatory T-cells, wherein figure 3a shows the percentage of regulatory T-cells in peribroncheal lymph-node cells (PBLN), and figure 3b) shows FACS-plot analyses of stained cells of a single animal per group; for a legend of figure 3a), see comments to figure 2;
figure 4 shows the interaction of a cystatin according to the present invention with CD36-expressing CHO-cells, wherein figure 4a is a schematic diagram of the assay used, and figure 4b is a representative analysis of the interaction of rAv17 with CD36 in comparison to CD36-negative cells,
figure 5 shows the sequences of cystatins from A. viteae, O. volvulus and two cystatins from C. elegans.

Moreover, reference is made to the following sequences, wherein SEQ ID NO:1 is the protein sequence of cystatin of Acanthocheilonema viteae (cystatin L43053),
SEQ ID NO:2 is the protein sequence of cystatin of Onchocerca volvulus (cystatin M37105), and SEQ ID NO:3 is the protein sequence of cystatin of Brugia malayi.

SEQ ID NO:1
L43053[Acanthocheilonema viteae]
MMLSIKEDGLLVVLLLSFGVTTVLVRCEEPANMESEVQAPNLLGGWQERNPEEKEIQDLLPKVLIKLNQL
SNVEYHLMPIKLLKVSSQVVAGLRYKMEIQVAQSECKKSSGEEVNLKTCKRLEGHPDQIITLEAWEKSWE
NFLQVKILEKKEVLSSV

SEQ ID NO:2
M37105 [Onchocerca volvulus]
MLTIKDGTLLIHLLLFSVVALVQLQGAKSARAKNPSKMESKTGENQDRPVLLGGWEDRDPKDEEILELLP
SILMKVNEQSNDEYHLMPIKLLKVSSQVVAGVKYKMDVQVARSQCKKSSNEKVDLTKCKKLEGHPEKVMT
LEVWEKPWENFMRVEILGTKEV

SEQ ID NO:3
AF177193_1 [Brugia malayi]
MMSTMSIKEGLLVILLSLFLFDTTALIHRREIPHMESKGQMQRGQVLLGGWQERSPEDNEILELLPSVLT
KVNQQSNDEYHLMPIKLLKVSSQVVAGVKYKMEVQVARSECKKSASEQVNLKTCKKLEGHPDQVMTLEVW
EKPWEDFLQVNILETKVLSSV

In the following, reference is made to the examples which are given to illustrate, not to limit the present invention.

### Examples

### Example 1

### Production of Cystatin from Nematodes

The cDNAs of *A. viteae-*cystatin (Av17, Hartmann, S., Kyewsky, B., Sonnenburg, B. & Lucius, R. (1997) A filarial cysteine protease inhibitor downregulates T cell proliferation and enhances IL-10 production. Eur. J. Immunol. 27, 2253 - 2260.), and of *O*. *volvulus*-cystatin (Ov17, Lustigman S, Brotman B, Huima T, Prince AM, McKerrow JH. (1992) Molecular cloning and characterization of onchocystatin, a cysteine proteinase inhibitor of Onchocerca volvulus. J Biol Chem. 267:17339-46.); Schönemeyer, A., Lucius, R., Sonnenburg, B., Brattig, N., Sabat, R., Schilling, K., Bradley, J. & Hartmann, S. (2001) Modulation of human T cell responses and macrophage functions by onchocystatin, a secreted protein of the filarial nematode Onchocerca volvulus. J. Immunol. 167: 3207 - 3215).
without the sequence encoding for the signal peptide were amplified by PCR using primers (Ov17: forward primer: 5'-gttcagttgcaaggagcc-3', reverse primer: 5'tcatacttcttttgttccc3'; Av17: forward primer: 5'gttttggtgcgctgtgaa3', reverse primer: 5'-tcacactgatgagagtac-3') derived from the full-length sequences. The PCR fragments were cloned into a T-overhang vector (pGEM-T Easy Vector Systems; Promega, Madison, WI) and further subcloned into the Eco-RI site of an expression vector yielding polypeptides with a leader of 6 histidines (pET-28 System; Novagen, Madison, USA). The plasmids were transformed into competent *E. coli* BL21 cells. Screening of transformants for expression was carried out by analysis of bacterial protein after induction with isopropylgalactoside (IPTG). The cell pellet of a 1.5 ml culture was analysed by SDS-PAGE, followed by staining with Coomassie blue. The recombinant protein was purified from the E. coli lysate using a glycerol-PBS-buffer (phosphate buffer saline (PBS) with 10% glycerol) by means of affinity chromatography using a nickel-NTA-column, and subsequently eluted from the column by a pH-change (from pH6 to pH5 to pH3). Subsequently, the eluted protein was dialyzed against PBS/0.05% Triton and sterile filtered.

### Example 2

### Sequence comparison

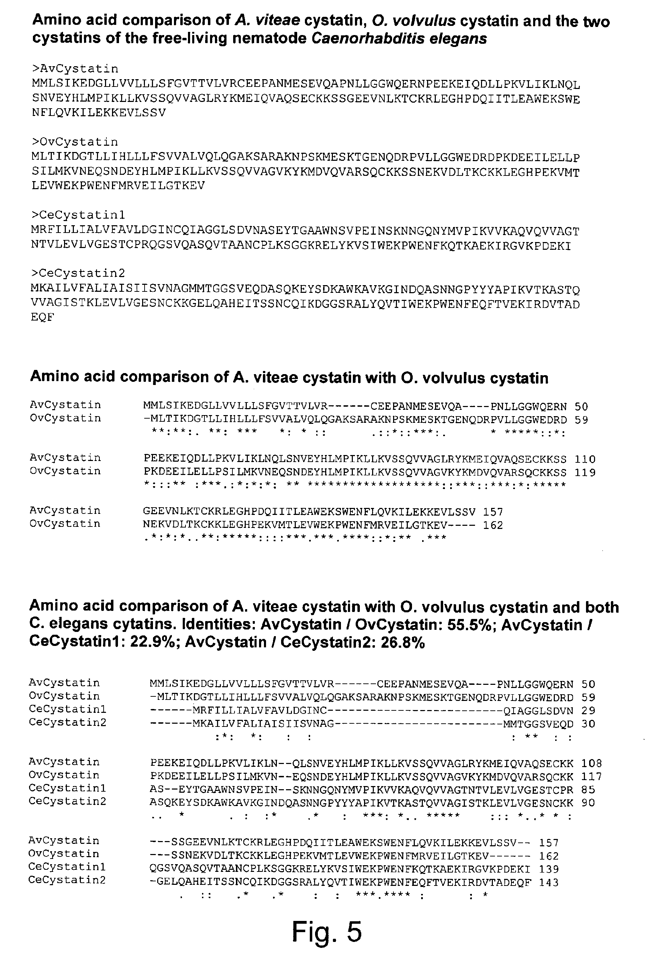

Figure 5 shows various cystatin sequences and an alignment between cystatin from Acanthocheilonema viteae and cystatin of Onchocerca volvulus, and a further alignment of the two aforementioned cystatins together with two cystatins (I + II) from the free-living nematode Caenorhabditis elegans. The identity between the first two cystatins is 55.5%, the identity between cystatin from Acanthocheilonema viteae and the two cystatins (I + II) from Caenorhabditis elegans is 22.9% and 26.8%, respectively. The identity between the cystatin of O. volvulus and cystatins I and II from C. elegans is 31.0% and 31.6%, respectively.

### Example 3

### Animal model for allergic airway inflammation

The following animal model served as a model for asthma with a severe airway inflammation: BALB/c-mice were sensitized twice using ovalbumin (OVA) (grade VI: Sigma Deisenhofen, Germany) (20 µg/200 µl PBS) in alum (2 mg). Fourteen days after the second sensitization using OVA, the mice were provoked intranasally using 100 µg OVA in 50 µl PBS on two subsequent days. Two days after the airway provocation, the function of the lungs was examined in vivo. One day later, the mice were sacrificed and a bronchoalveolar wash (BAL) was performed (Stock et al., 2004, European Journal of Immunology; 34:1817-1827). Subsequently, the lymphatic tissues were isolated. With respect to the administration of cystatin, the recombinantly produced protein (20 µg or 5 µg) was administered four times at an interval of one week, starting with the first sensitisation two hours prior to administration of the allergen (OVA). The nematode cystatin was administered intraperitoneally. At the time of airway provocation, no nematode cystatin was administered.

### The following parameters were determined:

Measurement of lung function: The lung function of the mice was measured using a provocation by inhalation with a rising dosage of bronchostringent metacholin, and was subsequently determined in a full body plethysmograph. The pressure difference between the plethysmograph chamber containing the animal and a reference chamber was measured. The pressure difference during the respiratory cycle of the animal can be indicated as enhanced pause using mathematical fomulae and can be taken as an index for respiratory constriction amongst the experimental animals (Hamelmann et al., 1997 Am. J. Respir. Crit. Care. Met.: 156:766-775).

Quantification of cells in bronchoalveolar fluid: Bronchoalveolar lavage was done twice by injecting 0,8ml PBS + protease inhibitors (complete™ Mini, Boehringer Mannheim Germany) into the lungs of each animal. The first lavage was centrifuged 10 minutes at 2200rpm (320g) at 4°C and the supernatant was removed and stored at -20°C for subsequent cytokine analysis. The second lavage was centrifuged at RT (2200rpm, 10min) and the cell pellets of both lavages were pooled and resuspended in 1ml PBS. 100µl of the cell suspension was centrifuged on a glass slide (using a cytospin centrifuge, 10min, 800rpm) creating a cell monolayer which was stained in fixing solution, eosin and thiazo-dye. Subsequently, the number of eosinophils, macrophages, lymphocytes and neutrophils was determined using histological standard protocols according to the manufacturers' instructions (Fisher Diagnostic Scientific, Schwerte, Germany).

Quantification of regulatory T-cells in peribroncheal lymph nodes and their cytokine production; using specific surface markers (CD4, CD25, CD103) and the expression of the transcription factor Foxp3, regulatory T-cells were determined using FACS (Lehmann et al, PNAS USA, 2002; 99:13031-13036). The production of cytokines (IL-4, IL-5, IL-10) of the cells of the bronchoalveolar wash and of the spleen was determined using ELISA, performed according to the manufacturers' instructions of BDE Biosciences using OptEIA™-Kits.

Total IgE and allergen-specific IgE-production: Total IgE-concentration as well as the OVA-specific IgE-concentration in serum was determined using ELISA. For determining the total IgE-titer, a sandwich ELISA was performed using an anti-IgE-antibody as a catcher-antibody, the sera of the experimental animals (diluted 1:100 in PBS/Tween) and biotinylated anti-IgE antibody as detection antibody. After incubation with strepavidin peroxidase (1:10,000), the reaction of the substrate TMB was photometrically detected at 460 nm. For determining the concentration, a commercially available IgE standard was used. The determination of the allergen-specific IgE was performed using the same method, however, the sera were diluted 1:2 and 1:10 and incubated using biotinylated allergen (50µl of 3µg/ml ovalbumine) as detection molecule. Streptavidinperoxidase (1:10,000) and TMB were used equivalently.

### Example 4

### Results of experiments

A treatment with recombinant A. viteae cystatin in a mouse model of allergic airway hyper reactivity demonstrates the influence of *A. viteae*-cystatin on allergic airway inflammation in mice (Figure 2). 5 mice per group were sensitized with ovalbumin (OVA) and treated with *A. viteae-*cystatin (Av17). Mice were sensitized twice with 20µg ovalbumin in alum (i.p.) and challenged 14 days after the second sensitization with 50µg ovalbumin in PBS (i.n.). 20 µg cystatin or 5µg cystatin were applied 4 times in weekly intervals during the time of sensitization. Sensitization and challenge with OVA led to a significant increase in total cell number reflected by the numbers of eosinophils in the bronchoalveolar fluid. The concurrent treatment with 20µg/ml A. viteae cystatin completely abolished the effect of OVA on eosinophils (Fig. 2b). Such a strong influence could not be seen by a lower dosage of cystatin (5µg/ml). A second feature of an allergic airway inflammation is the significant upregulation of the allergen-specific IgE concentration. Again, as shown in figure 2c, the inventors revealed that sensitization and challenge with OVA significantly induced the production of OVA-specific IgE in sera of mice. However, treatment with cystatin completely reversed the impact on allergen-specific IgE. Another prominent characteristic of an airway hyperreactivity is the upregulation of IL-5. Here, the inventors could show that mice sensitized and challengend with OVA showed high levels of IL-5 in the bronchoalveolar fluid, however animals treated simultaneously with cystatin did not show such an increase in IL-5 production. In contrast, the IL-5 values of cystatin treated mice were comparable to the levels of naïve control mice (Fig. 2d). Together, these data indicate that repeated treatment with 20 ug cystatin reduces allergic airway inflammation to the level of a healthy individual.

The panels A - D of figure 2 show the following: A: SDS-gel of purified recombinant *A. viteae*-cystatin; B: Numbers of eosinophils in the bronchoalveolar fluid (BALF). C: Serum levels of OVA-specific IgE. D: Production of IL-5 in BALF. Naive: PBS-treated mice; OVA: ovalbumin-treated mice; Av17: Av17/OVA-treated mice with 20 or 5 µg Av17.

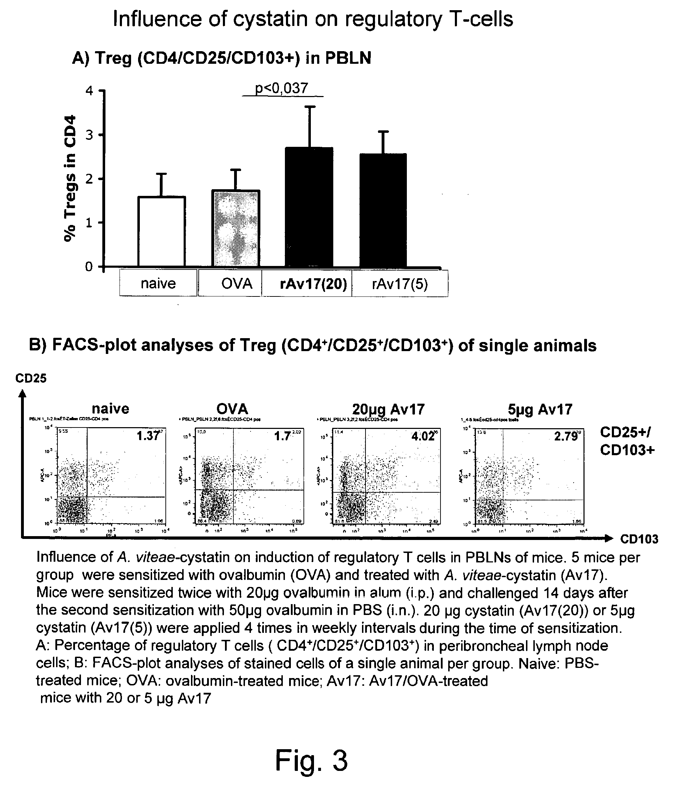

Furthermore, A. viteae-cystatin showed an influence on induction of regulatory T cells in PBLNs of mice (Figure 3). Again, 5 mice per group were sensitized with ovalbumin (OVA) and treated with *A. viteae*-cystatin (Av17). Mice were sensitized twice with 20µg ovalbumin in alum (i.p.) and challenged 14 days after the second sensitization with 50µg ovalbumin in PBS (i.n.). 20 µg cystatin or 5µg cystatin were applied 4 times in weekly intervals during the time of sensitization. Regulatory T cells (Tregs) were characterized by staining of their cell surface markers CD25 and CD 103 and double positive cells were 98% Foxp3 positive, a transcription factor, which represents another reliable marker for Tregs. The analyses showed that sensitization of mice with OVA and the subsequent challenge with OVA did not lead to an increase in regulatory T cells in comparison to naive mice. But the animals, which were concurrently treated with cystatin showed a significant increase in regulatory T cells. Figure 3a shows the mean values of Tregs of all animals per group (n=5). Figure 3b show a representaive FACS-plot analysis of a single animal. On the upper right panel of each plot is the percentage of cells shown which were double positive for both Treg markers. These data clearly show that the application of cystatin led to an increase in the number of these potent suppressor cells.

The panels A - B of figure 3 show: A: Percentage of regulatory T cells (CD4+/CD25+/CD103+) in peribroncheal lymph node cells; B: FACS-plot analyses of stained cells of a single animal per group. Naive: PBS-treated mice; OVA: ovalbumin-treated mice; Av17: Av17/OVA-treated mice with 20 or 5 µg Av17.

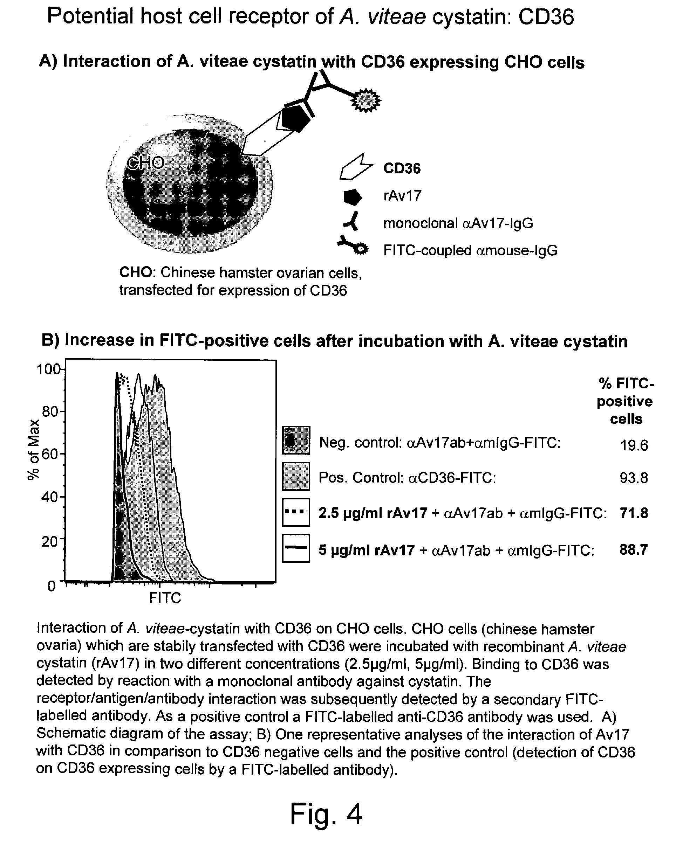

In addition, the inventors have evidence for the interaction of *A. viteae*-cystatin with the scavenger receptor CD36(Figue 4). CHO cells (chinese hamster ovaria), which are stabily transfected with CD36 were incubated with recombinant *A. viteae* cystatin (rAv17) in two different concentrations (2.5µg/ml, 5µg/ml). Binding to CD36 was detected by reaction with a monoclonal antibody against cystatin. The receptor/antigen/antibody interaction was subsequently detected by a secondary FITC-labelled antibody. As a positive control a FITC-labelled anti-CD36 antibody was used. The analyses showed that cystatin bound to CD36, which could be detected by the significant increase in fluorescence positive cells from 20% to 71% or 89 % respectively (Fig. 4). An interaction of cystatin with the scavenger receptor CD36 on immune cells such as macrophages could explain its therapeutic potential, as targeting of CD36 results in the production of IL-10 and other anti-inflammatory processes.

Figure 4 A - B shows:
(A) Schematic diagram of the assay; B) One representative analyses of the interaction of Av17 with CD36 in comparison to CD36 negative cells and the positive control (detection of CD36 on CD36 expressing cells by a FITC-labelled antibody).

Figure 5 shows a sequence comparison of various cystatins. Amino acid comparison of A. viteae and O. volvulus cystatin with the cystatins of the free living nematode C. elegans shows an identity of 56% between the parasitic cystatins in comparison to about 30% to the cystatins of the free-living nematode. Differences in the amino acid sequence of the cystatins of parasitic nematodes in comparison to the free-living nematodes imply functional differences due to specific protein domains.

The features of the present invention disclosed in the specification, the claims and/or in the accompanying drawings, may, both separately, and in any combination thereof, be material for realizing the invention in various forms thereof.

## Claims

1. Use of a cystatin derived from a nematode for the manufacture of a medicament for the prevention and/or treatment of an allergic and/ or autoimmune disease in a patient.

2. Use according to claim 1, **characterized in that** said cystatin is derived from a parasitic nematode.

3. Use according to claim 2, **characterized in that** said parasitic nematode is parasitic to humans.

4. Use according to any of claims 1-3, **characterized in that** said nematode is selected from the group comprising Onchocerca volvulus, Brugia malayi, Wuchereria bancrofti, Loa loa and Acanthocheilonema viteae.

5. Use according to any of the foregoing claims, **characterized in that** said allergic and/ or autoimmune disease is selected from the group comprising allergic diseases of the respiratory organs, such as asthma, hay fever, allergic sinusitis, allergic rhinitis, of the gastrointestinal system, such as food allergies, of the skin, such as atopic dermatitis, systemic allergic diseases, such as anaphylactic reactions, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

6. Use according to claim 5, **characterized in that** said allergic disease is asthma or hay fever.

7. Use according to any of the foregoing claims, **characterized in** said cystatin has a sequence selected from the group comprising SEQ ID NO:1 (Acanthocheilonema viteae cystatin L43053),
SEQ ID NO: 2 (Onchocerca volvulus cystatin M37105) and SEQ ID NO:3 (Brugia malayi cystatin) and sequences that are 70% identical, preferably 80% identical, more preferably 90% identical and, most preferably, 99% identical to any of the foregoing.

8. Use according to any of the foregoing claims, **characterized in that** said cystatin is recombinant cystatin and has been produced by a procaryotic or eucaryotic expression system.

9. Use according to any of the foregoing claims, **characterized in that** said disease is associated with an increased count of eosinophil blood cells and/or with an increased level of IgE, when compared with a patient not having said disease, and said medicament, upon its administration to said patient, leads to a reduction of said increased count of eosinophil blood cells and/or to a reduction of said increased level of IgE, preferably to a count of eosinophil blood cells and/or to a level of IgE of a healthy individual.

10. Use according to claim 9, **characterized in that** said increased count of eosinophil blood cells is > 4% of all white blood cells of a patient or > 360/µl total number of eosinophil blood cells in peripheral blood of a patient, and said increased level of IgE is > 100 kU/l serum level in an adult patient.

11. Use according to any of the foregoing claims, **characterized in that** said cystatin is administered to said patient as a protein.

12. Use according to any of claims 1 - 10, **characterized in that** said cystatin is administered to said patient as a nucleic acid encoding said cystatin.

13. Use according to any of the foregoing claims, **characterized in that** said cystatin is administered systemically to said patient, preferably by injection, inhalation and or other incorporation such as ingestion.

14. Use according to any of the foregoing claims, **characterized in that** said cystatin is administered to said patient intranasally, intrapulmonarily, intraperitoneally, intrathecally, intralesionally, subcutaneously and/or intramuscularly.

15. Use according to any of the foregoing claims, **characterized in that** said cystatin is administered in combination with another drug selected from the group of anti-inflammatory drugs, such as corticosteroids, non-steroidal anti-inflammatory drugs, and/or anti-histamines.

16. Use according to any of the foregoing claims, **characterized in that** said patient is a mammal, preferably a human being.

17. Use according to any of the foregoing claims, **characterized in that** said cystatin is used to bind to CD36 receptor.

18. A method of screening for a candidate drug useful for the prevention and/or treatment of an allergic and/or autoimmune disease comprising the following steps:
- providing a first group of cells of a type expressing CD36-receptor,
- exposing said cells to a cystatin derived from a nematode, said cystatin and said nematode being defined as in any of claims 1-16,
- detecting and quantitating, as a first signal, the extent of binding between said cystatin and said CD36 receptor,
- providing a second group of cells of the same type as the first group of cells,
- exposing said second group of cells to a candidate compound,
- detecting and quantitating, as a second signal, the extent of binding between said candidate compound and said CD36 receptor,
- comparing said first signal with said second signal, and
- identifying said candidate compound as a candidate drug for the prevention and/or treatment of an allergic and/or autoimmune disease, if the extent of binding quantitated by said second signal is equal to or greater than the extent of binding quantitated by said first signal.

19. Use of CD36-receptor in a method of screening for candidate compounds for the prevention and/or treatment of an allergic and/or autoimmune disease is a patient.
